Europäisches Patentamt

European Patent Office

Office européen des brevets

(11) Publication number: **0 169 562**
**A1**

(12) **EUROPEAN PATENT APPLICATION**

(21) Application number: 85109326.0

(22) Date of filing: 25.07.85

(51) Int. Cl.⁴: **C 07 K 15/14**
C 12 N 15/00, C 12 P 21/00
A 61 K 37/02, A 61 K 35/14
A 61 K 35/16
//C12R1:19, C12R1:125

(30) Priority: 27.07.84 US 634933

(43) Date of publication of application:
29.01.86 Bulletin 86/5

(84) Designated Contracting States:
AT BE CH DE FR GB IT LI LU NL SE

(71) Applicant: MELOY LABORATORIES, INC.
6715 Electronic Drive
Springfield Virginia 22151(US)

(72) Inventor: Drohan, William N.
5232 Perth Court
Springfield Virginia(US)

(72) Inventor: Lee, Sally S.G.
14960 Carry Back Drive
Gaithersburg Maryland(US)

(72) Inventor: Ricca, George A.
7903 Foote Lane
Springfield Virginia(US)

(74) Representative: Patentanwälte Grünecker, Kinkeldey,
Stockmair & Partner
Maximilianstrasse 58
D-8000 München 22(DE)

(54) Recombinant factor VIII-R.

(57) Human Factor VIII-R essentially free of other proteins of human origin is disclosed. Characteristically, the Factor VIII-R protein is glycosylated. The Factor VIII-R is produced by recombinant DNA techniques in host cells or other self-replicating systems and is provided in essentially pure form. Also provided are methods and compositions for preparing the above-described Factor VIII-R as well as therapeutic compositions and uses for the Factor VIII-R protein in the treatment of coagulation disorders in humans and animals. The invention further provides replicable expression vectors incorporated with a DNA sequence encoding human Factor VIII-R and host cell or cell-free self-replicating systems transformed or transfected thereby.

./...

Croydon Printing Company Ltd.

## RECOMBINANT FACTOR VIII-R

This invention relates generally to the field of structural gene cloning and the use of such genes in the recombinant DNA directed synthesis of desired protein products. More particularly, it relates to a new Factor VIII protein (Factor VIII-R), its recombinant DNA directed synthesis and use in the treatment of coagulation disorders, such as Von Willebrand's disease.

In general, recombinant DNA techniques have now become well known. See Methods in Enzymology, (Academic Press) volumes 65 and 68 (1979); 100 and 101 (1983) and the references cited therein, all of which are incorporated herein by reference. An extensive technical discussion embodying most commonly used recombinant DNA methodologies can be found in Maniatis, et al, Molecular Cloning, Cold Spring Harbor Laboratory (1982). Genes coding for various polypeptides may be cloned by incorporating a DNA fragment coding for the polypeptide in a recombinant DNA vehicle, e.g., bacterial or viral vectors, and transforming a suitable host, typically an Escherichia coli (E.coli) cell line, and isolating clones incorporating the recombinant vectors. Such clones may be grown and used to produce the desired polypeptide on a large scale.

Several groups of workers have isolated mixtures of mRNA from eukaryotic cells and employed a series of three enzymatic reactions to synthesize double-stranded DNA copies of entire genes which are complementary to this mRNA mixture. In the first reaction, mRNA is transcribed to form a single-stranded complementary DNA (cDNA) by an RNA-directed DNA polymerase, also called reverse transcriptase. Reverse transcriptase synthesizes DNA in the 5' - 3' direction, utilizes deoxyribonucleoside 5'-triphosphates as precursors, and require both a template and a primer strand, the latter of which must have a free 3'-hydroxyl terminus. Reverse transcriptase products, whether partial or complete copies of the mRNA template, often possess short, partially double-stranded hairpins ("loops") at their 3' termini. In the second reaction, these "hairpin loops" can be exploited as primers for DNA polymerases. Preformed DNA is required both as a template and as a primer in the action of DNA polymerase. The DNA polymerase requires the presence of a DNA strand having a free 3'-hydroxyl group, to which new nucleotide residues are added to extend the chain in the 5' - 3' direction. The products of such sequential reverse transcriptase and DNA polymerase reactions still possess a loop at one end. The apex of the loop or "fold-point" of the double-stranded DNA, which has thus been created, is substantially a single-strand segment. In the third reaction, this single-strand segment is cleaved with the single-strand specific nuclease S1 to generate a "blunt-end" duplex DNA segment. This general method is applicable to any mRNA mixture, and is described by Buell, et al, J. Biol. Chem, 253:2483 (1978).

The resulting double-stranded cDNA (ds-cDNA) is inserted into cloning vehicles by any one of many known techniques, depending at least in part on the particular vehicle being used. Various insertion methods are discussed in considerable detail in Methods In Enzymology, 68:16-18, and the references cited therein.

These cloning vehicles usually impart an antibiotic resistance trait on the host. Once the DNA segment is inserted, the cloning vehicle is used to transform a suitable host. Such hosts are generally prokaryotic or eukaryotic cells. At this point only a few of the transformed or transfected hosts contain the desired cDNA. The sum of all transformed or transfected hosts constitutes a gene "library". The overall ds-cDNA library created by this method provides a representative sample of the coding information present in the mRNA mixture used as the starting material.

If an appropriate oligonucleotide sequence is available, it can be used to identify clones of interest as follows. Individual transformed or transfected cells are grown as colonies on nitrocellulose filter paper. These colonies are lysed, the DNA so-released is covalently attached to the filter paper by heating and the sheet is incubated with a labeled oligonucleotide probe. The probe sequence is complementary to the structural gene of interest. The probe hybridizes with the ds-cDNA for which it is complementary and this is identified by autoradiography. The clones are characterized to identify a clone containing all the structural information for the desired protein. The nucleic acid sequence coding for the protein of interest is isolated and reinserted into an expression vector. The expression vector brings the cloned gene under the regulatory control of a specific prokaryotic or eukaryotic control element which allows the efficient expression (transcription and translation) of the cloned full length ds-cDNA. Thus, this general technique is only applicable to those proteins for which at least a portion of their amino acid or DNA sequence is known and for which an oligonucleotide probe is available. See, generally, Maniatis, et al, supra.

More recently, methods have been developed to identify specific clones by proving bacterial colonies with antibodies specific for the encoded protein of interest. This method can

0169562

only be used with "expression vector" cloning vehicles since elaboration of the product protein is required. The structural gene is inserted into the vector adjacent to regulatory gene sequences that induce expression of the protein. The cells are lysed, either by the vector or by chemical methods, and the protein detected by the specific antibody and a labeling system, e.g., enzyme immunoassay. An example of this is the lambda gt11 system described by Young and Davis, Proc. Nat'l. Acad. Sci. USA, 80:1194-1198 (1983) and Young and Davis, Science, 22:778 (1983).

Normal human plasma contains a complex of two proteins which is referred to as the Factor VIII complex. One component of the Factor VIII complex influences platelet adhesiveness and is designated Factor VIII-R. A deficiency in Factor VIII-R is characteristic of Von Willebrand's disease, an autosomally inherited disease.

There is little information in the literature about the biochemistry of Factor VIII-R. Most studies of Factor VIII-R have been performed using the intact Factor VIII complex. Recently, Factor VIII-R has been separated from the Factor VIII complex and most other plasma proteins, principally by cryoprecipitation, immuno-adsorption, ion exchange chromatography and gel filtration. In general, see Hoyer, Blood, 58:1:13 (1981) and Zimmerman, et al, U.S. Patent No. 4,361,509, Zimmerman, et al, Prog. in Hematol., 13:279 (1983) and the references cited in each.

Factor VIII-R has therapeutic value because of its ability to correct the clotting deficiency in Von Willebrand's patients. Unfortunately, the available methods of securing Factor VIII-R are limited to fractionation of human plasma, as described above. Producing Factor VIII-R by plasma fractionation provides only limited quantities which vary from preparation to preparation, are expensive and subject the recipient to the risk of acquiring diseases such as hepatitis and acquired immune deficiency syndrome.

0169562

In recognizing and overcoming the above-described problems, the present invention has made it possible to provide readily available large quantities of Factor VIII-R Von Willebrand's factor which is free of the disease transmission risks of plasma fractionation preparations. This has been achieved with oligonucleotides coding specifically for segments of the Factor VIII-R protein molecule, the application of recombinant DNA technology to preparing cloning vehicles encoding for the Factor VIII-R protein, and screening/isolating procedures for recovering human Factor VIII-R protein essentially free of other proteins of human origin.

Accordingly, the present invention provides human Factor VIII-R essentially free of other proteins of human origin. Characteristically, the Factor VIII-R protein is glycosylated. The Factor VIII-R is produced by recombinant DNA techniques in host cells or other self-replicating systems and is provided in essentially pure form. Also provided are methods and compositions for preparing the above-described Factor VIII-R as well as therapeutic compositions and uses for the Factor VIII-R protein in the treatment of coagulation disorders in humans and animals.

The invention further provides replicable expression vectors incorporated with a DNA sequence encoding human Factor VIII-R and host cell or cell-free self-replicating systems transformed or transfected thereby. The host system is usually of prokaryotic, e.g. E.coli or B.subtilis, or eukaryotic cells.

The human Factor VIII-R is produced by a process which comprises (a) preparing a replicable expression vector capable of expressing the DNA sequence encoding human Factor VIII-R in

-6-

0169562

a suitable host cell or cell-free replicating system; (b) transforming said host system to obtain a recombinant host system; (c) maintaining said recombinant host system under conditions permitting expression of said Factor VIII-R encoding DNA sequence to produce human Factor VIII-R protein; and (d) recovering said human Factor VIII-R protein. Preferably, the Factor VIII-R encoding replicable expression vector is made by preparing a double-stranded complementary DNA (ds-cDNA) preparation representative of a messenger RNA pool containing messenger RNA for Factor VIII-R and incorporating DNA from the ds-cDNA pool into replicable expression vectors. The preferred mode of recovering the human Factor VIII-R comprises reacting the proteins expressed by the recombinant host system with a reagent composition comprising at least one binding protein specific for Factor VIII-R, observing any detectable response therefrom and isolating the identified Factor VIII-R from the host system.

0169562

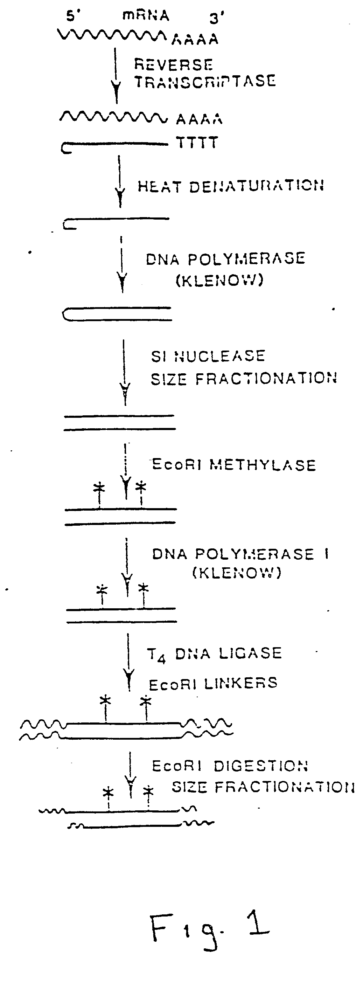

In the accompanying drawings, Fig. 1 is a flow sheet showing the enzymatic reactions described in the Example.

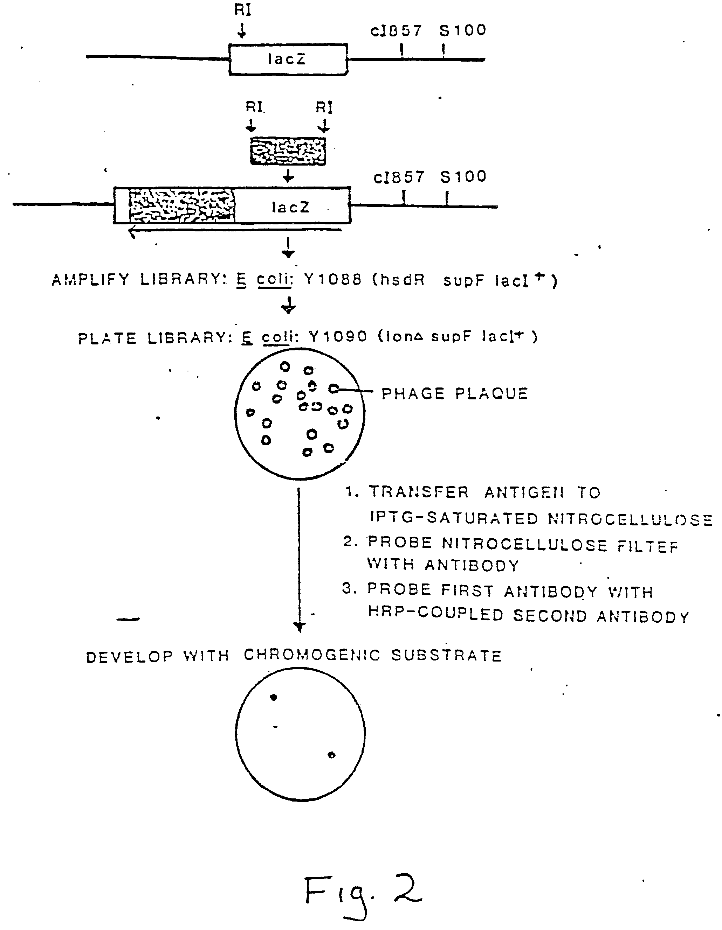

Fig. 2 is a flow sheet showing the procedures used in preparing the recombinant vector, incorporating it into a host and screening a library for identification of the desired clone(s) which were used in the Example.

0169562

As used herein, "human Factor VIII-R" denotes human Factor VIII-R produced by cell or cell-free culture systems, in bioactive forms having the capacity to participate in normal hemostasis as does Factor VIII-R native to human plasma.

Different alleles of Factor VIII-R can exist in nature. These variations may be characterized by difference(s) in the overall nucleotide sequence of the structural genes coding for proteins of identical biological function. In addition, the location and degree of glycosylation as well as other post-translational modifications may vary and will depend to a degree upon the nature of the host and environment in which the protein is produced. It is possible to produce human Factor VIII-R analogs having single or multiple amino acid substitutions, deletions, active fragments, additions or replacements. These can include analogs which have been provided with additional sites for platelet binding as well as for specificities different from those of endogenous or native human Factor VIII-R. Subgenomic fragments, including those embodying specific desired biological properties can also exist or be prepared. All such allelic variations, modifications and analogs resulting in derivatives of human Factor VIII-R which retain the biological properties of native human Factor VIII-R are included within the scope of this invention.

Expression vectors refer to vectors which are capable of transcribing and translating DNA sequences contained therein, where such sequences are linked to other regulatory sequences capable of effecting their expression. These expression vectors must be replicable in the host organisms or systems either as episomes, bacteriophage, or as an integral part of

0169562

the chromosomal DNA. One form of expression vector which is particularly suitable for use in the invention is the bacteriophage, viruses which normally inhabit and replicate in bacteria. Particularly desirable phage for this purpose are the lambda gt10 and lambda gt11 phage described by Young, et al, _supra_. The lambda gt11 is a general recombinant DNA expression vector capable of producing polypeptides specified by the inserted DNA. The recombinant vector can be propagated in its host cell as a single copy genomic insert to enhance its stability. This vector responds to induction with a rapid increase in copy number and high level transcription of the foreign DNA. To minimize degradation, foreign eukaryotic protein polypeptides are synthesized as a fusion of the eukaryotic moiety from its structural DNA sequence with all but a small portion of the prokaryotic protein B-galactosidase from its gene. The use of host cells defective in protein degradation pathways may also increase the lifetime of novel proteins produced from the induced lambda gt11 clones. Proper expression of foreign DNA In lambda gt11 clones will depend upon the orientation and reading frame of the inserted DNA with respect to the B-galactosidase promoter. Another form of expression vector useful in recombinant DNA techniques is the "plasmid" -a circular, unintegrated extrachromosomal double-stranded DNA loop. The invention includes any other form of expression vector which serves an equivalent function and which has or subsequently becomes known in the art.

Recombinant vectors and methodology disclosed herein are suitable for use in host cells over a wide range of prokaryotic and eukaryotic organisms. In general, of course, prokaryotes are preferred for cloning DNA sequences and in constructing the vectors useful in the invention. For example, _E.coli_ K12 strain MM294 (ATCC No. 31446) is particularly useful. Of course, other microbial strains may be used. Vectors containing replication and control sequences which are derived from species compatible with the host cell or system are used in connection with these hosts. The vector ordinarily carries an origin or replication, as well as characteristics capable of providing phenotypic selection in transformed cells. E.coli can be transformed using pBR322, a plasmid derived from an E.coli species (Bolivar, et al, Gene, 2:95 (1977). The pBR322 contains genes for ampicillin and tetracycline resistance, consequently providing a means for identifying transformed cells. The expression vector must also contain promoters which can be used by the vector for expression of its own proteins. Common prokaryotic promoters include the beta lactamase (penicillinase), lactose, tryptophan (trp), and the pR and pL promoters of the bacteriophage lambda. Combinations of these promoters have also been used (e.g., TAC, which is a fusion of the trp promoter with the lactose operator). Other promoters have also been discovered and utilized, and details concerning their nucleotide sequences have been published enabling a skilled worker to ligate them functionally with appropriate vectors.

In addition to prokaryotes, eukaryotic microbes, such as yeast cultures, may also be used. Saccharomvcles cerevisiae, or common baker's yeast is the most commonly used among eukaryotic microorganisms, although a number of other strains are commonly available. Suitable promoting sequences in yeast vectors include the promoters for 3-phosphoglycerate kinase or other glycolytic enzyme systems. In constructing suitable expression vectors, the termination sequences associated with these genes are also ligated into the expression vector 3' of the sequence desired to be expressed to provide polyadenylation of the mRNA and termination. Any vector containing a yeast-compatible promoter, origin of replication and appropriate termination sequence is suitable for expression of Factor VIII-R.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years. Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promoters are derived from polyoma, Adenovirus 2, and, most frequently, Simian Virus 40 (SV40). Further, it is also possible, and often desirable, to utilize promoter or control sequences naturally associated with the desired gene. sequence, provided such control sequences are compatible with the host system. To increase the rate of transcription, eukaryotic enhancer sequences can also be added to the construction. These sequences can be obtained from a variety of animal cells or oncorna viruses such as the mouse sarcoma virus.

An origin of replication may be provided either by construction of the vector to include an exogenous origin, such as that provided by SV40 or other viral sources, or may be provided by the host cell chromosomal replication mechanism. If the vector is integrated into the host cell chromosome, the latter is often sufficient.

Host cells can prepare human Factor VIII-R proteins which can be of a variety of chemical compositions. The protein is produced having methionine as its first amino acid (present by virtue of the ATG start signal codon inserted in front of the structural gene). The methionine may also be intra or extracellularly cleaved, having its normally first amino acid. The protein may be produced together with either its signal polypeptide or a conjugated protein other than the conventional signal polypeptide, the signal polypeptide of conjugate being specifically cleavable in an intra or extracellular environment. Finally, Factor VIII-R may be produced by direct expression in mature form without the necessity of cleaving away any extraneous polypeptide.

Recombinant host cells refers to cells which have been transformed with vectors constructed using recombinant DNA techniques. As defined herein, Factor VIII-R is produced as a consequence of this transformation. Factor VIII-R produced by such cells is referred to as "recombinant Factor VIII-R".

B.   Recombinant and Screening Methodology

The procedures below are but some of a wide variety of well established procedures to produce specific reagents useful in the process of this invention. The general procedure for obtaining a messenger RNA (mRNA) mixture is to prepare an extract from a tissue sample or to culture cells producing the desired protein and to extract the mRNA by a process such as that disclosed by Chirgwin, et al, Biochemistry, 18:5294 (1979). The mRNA is enriched for poly(A) mRNA-containing material by chromatography on oligo (dT) cellulose or poly (U) sepharose, followed by elution of the poly(A) containing mRNA-enriched fraction.

The above poly(A) containing mRNA-enriched fraction is used to synthesize a single-strand complementary DNA (ss-cDNA) using reverse transcriptase. As a consequence of DNA synthesis, a hairpin loop is formed at the 3' end of the DNA which will initiate second strand DNA synthesis. Under appropriate conditions, this hairpin loop is used to effect synthesis of the second strand in the presence of DNA polymerase and nucleotide triphosphates.

The resultant double-strand cDNA (ds-cDNA) is inserted into the expression vector by any one of many known techniques. General methods, etc., can be found in Maniatis, supra and Methods in Enzymology, vol. 65 and 68 (1980); 100 and 101 (1983). In general, the vector is linearized by at least one restriction endonuclease, which wil produce at least two blunt ends or cohesive ends. The DNA is ligated with or joined to the vector insertion site.

If prokaryotic cells or cells which contain substantial cell wall construction are used, the most common method of transformation with the expression vector is calcium chloride pretreatment as described by Cohen, F.N., et al, Proc. Nat'l. Acad. Sci.(USA), 69:2110 (1972). If cells without cell wall barriers are used as host cells, transfection is carried out by the calcium phosphate precipitation method described by Graham and Ver der Eb, Virology, 62:546 (1978). Other methods for introducing DNA into cells, such as nuclear injection or protoplast fusion, have also been successfully used. The organisms are then cultured on selective media and proteins for which the recombinant cDNA library encodes are produced.

Clones containing part or the entire sequence of Factor VIII-R can be identified by hybriding the ds-cDNA mixture so formed with a chemically synthesized oligonucleotide probe. The oligonucleotide probe is determined from the primary amino acid sequence of the Factor VIII-R protein. The sequence of the oligonucleotides which code for a given amino acid sequence can be deduced based on the degenerate nature of the genetic code.

0169562

Clones are grown on nitrocelulose filters, and prepared for hybridization according to Grunstein and Hogness, Proc. Nat. Acad. Sci., 72:3961 (1975). The filters are reacted with 32p-labeled oligonucleotides synthesized based on the amino acid sequence of Factor VIII-R and then washed, dried, and autoradiographed. Clones that showed a strong hybridization signal are selected for further analysis. DNA can then be isolated by the method described in Norgard, et al, J. Bacteriol., 138:270 (1979), cleaved with restriction endonuclease PstI and the cleavage fragments separated by electrophoresis in horizontal 1.5% agarose gels,. as described in Maniatis, supra (1975). Vectors containing the longest cDNA inserts can then be sequenced as described by Maxam and Gilbert, Methods in Enzymology, 65:499 (1980). The clone(s) which correspond to the entire nucleotide sequence coding for Factor VIII-R are identified.

Clones containing part or the entire gene for Factor VIII-R are also identified with specific antibodies directed against part or all of the Factor VIII-R protein. This method of identification requires that the ds-cDNA be inserted into a vector containing appropriate regulatory nucleic acid sequences adjacent to the insertion site. These regulatory sequences initiate transcription and translation of those ds-cDNA molecules inserted in the vector. Those clones containing Factor VIII-R cDNA sequences correctly positioned relative to the regulatory sequences proliferate part or all of the Factor VIII-R protein. This Factor VIII-R amino acid sequence is detected by appropriately specific antibodies. Such a cloning system is the lambda gt11 system first described by Young and Davis, supra.

Specific binding assay screening techniques are preferably used to identify Factor VIII-R protein which has been elaborated by the transformed host. Specific binding assays are based on the specific interaction between a ligand, i.e., a bindable analyte under determination, and a binding partner therefor. Where, as in the case of Factor VIII-R, one of the ligand and its binding partner is an antigen and the other is a corresponding antibody, the assay is known as an immunoassay.

Several different binding assay systems are known in the art, and are generally categorized according to the nature of the label used. The affected characteristic of the label may be of any measurable nature. In the majority of cases, the system will incorporate homogeneous specific binding assay reagents which interact with the ligand in or its binding capacity of the sample in an immunochemical manner. For example, in one system, the label is an enzyme and the ability of the enzyme to act on the substrate is affected, either in a positive or negative sense, by binding of the label conjugate with its binding partner. Action of the enzyme on the substrate produces a product that is distinguishable in some feature, usually fluorescence or light absorption (color).

## EXAMPLE

### A.  Growth of Endothelial Cells

Human umbilical vein endothelial cells were grown as described by Maciag, et al, J. Cell Biol. 91:420 (1981). They were cultured in Medium 199 containing 20% fetal calf serum, endothelial cell growth factor (100 ug/ml), penicillin G (10 u/ml), streptomycin (1 ug/ml), and fungizone (5 ug/ml). The cells were fed every 2-3 days until confluent. At confluence, the cells were harvested by scraping into PBS.

### B.  Preparation of Total RNA

Total RNA (messenger, ribosomal and transfer) was extracted from the endothelial cells essentially as described by Chirgwin, supra, (1979). The cells were homogenized in 15 volumes of a solution containing 4 M guanidine thiocyanate, 25 mM sodium citrate at pH 7.0, 0.5% N-laurylsarcosine, 0.1 M 2-mercaptoethanol, and 0.2% Antifoam A (Sigma Chemical Co., St. Louis, MO). The homogenate was centrifuged at 6,000 rpm in a Sorvall GSA rotor for 15 minutes at $10^{\circ}$ C. The supernatant fluid was adjusted to pH 5.0 by addition of acetic acid and the RNA precipitated by 0.75 volumes of ethanol at $-20^{\circ}$ C for two hours. RNA was collected by centrifugation and dissolved in 7.5 M guanidine hydrochloride containing 25 mM sodium citrate and 5 mM dithiothreitol. Following two additional precipitations using 0.5 volumes of ethanol, the residual guanidine hydrochloride was extracted from the precipitate with absolute ethanol. RNA was dissolved in sterile water, insoluble material removed by centrifugation, and the pellets were reextracted with water. The RNA was adjusted to 0.2 M potassium acetate and precipitated by addition of 2.5 volumes of ethanol at $-20^{\circ}$ C overnight.

C. Preparation of Poly(A)-containing RNA

The total RNA precipitate, prepared as described above, was dissolved in 20 mM Hepes buffer at pH 7.2 containing 10 mM EDTA and 1% SDS, heated at 65° C for 10 minutes, then quickly cooled to 25° C. The RNA solution was then diluted with an equal volume of water and NaCl was added to bring the final concentration to 300 mM NaCl. Samples containing up to 2400 $A_{260}$ units of RNA were chromotographed on poly(U)-sepharose using standard procedures. Poly(A)-containing RNA was eluted with 70% formamide containing 1 mM Hepes buffer (pH 7.2), and 2 mM EDTA. The eluate was adjusted to 0.24 M NaCl and the RNA was precipitated by 2.5 volumes of ethanol at -20° C.

D. Construction of cDNA Clones in Lambda gt11

The procedures followed for all enzymatic reactions have been generally diagrammed in Fig. 1. The mRNA (20 ug) was copied into ds-cDNA with reverse transcriptase and DNA polymerase I exactly as described by Buell, et al, supra, and Wikens, et al J. Biol. Chem., 253:2483 (1978). The ds-cDNA was desalted on Sephadex G-50 and the void volume fractions further purified on an Elutip-D column, (Schleicher & Schuell, Keene, NH), following the manufacturer's directions. The ds-cDNA was made blunt-ended by incubation with S1 nuclease, Norgard, et al, supra. The reaction mixture consisted of 0.2 M sodium acetate (pH 4.5), 0.4 sodium chloride, 2.5 mM zinc acetate and 0.1 unit of S1 nuclease per ng of ds-cDNA, made to a final reaction volume of 100 ul. The ds-cDNA was incubated at 37° C for one hour, extracted with phenol:chloroform, and then desalted on a Sephadex G-50 column as described above.

The double-stranded cDNA was then treated with Eco RI methylase and DNA polymerase I (Klenow) using reaction conditions described in Maniatis, Molecular Cloning, surpa. The

0169562

cDNA was again desalted on Sephadex G-50 as described above and then ligated to 0.5 ug of phosphorylated Eco RI linkers with $T_4$ DNA ligase (Maniatis, _supra_). The mixture was then cleaved with Eco RI and fractionated on an 8% acrylamide gel in Tris-Borate buffer (Maniatis, _supra_). DNA with a size greater than 1 kilobase was eluted from the gel and recovered by binding to an Elutip-D column, eluted with 1 M NaCl and then collected by ethanol precipitation.

The procedures followed in the remainder of this example have been generally diagrammed in Fig. 2. The DNA fragments were then inserted into Eco RI, cleaved and phosphatase-treated lambda gt11 with $T_4$ DNA ligase to produce a library of approximately eight (8) hundred thousand recombinant phage. The library was amplified by producing plate stocks at $42^{\circ}$ C on _E.coli_ Y1088 [supE supF metB trpR hsdR⁻hsdM⁺ tonA21 strA lacU169 proC::Tn5 (pMC9)]. Amplification procedures are described in Maniatis, _supra_. Important features of this strain, described by Young and Davis, include supF (required for suppression of the phage amber mutation in the S gene), hsdR⁻ hsdM⁺ (necessary to prevent restriction of foreign DNA prior to host modification), lacU169 (a deletion of the lac operon reduces host-phage recombination and is necessary to distinguish between lambda gt11 recombinants (generally little or no B-galactosidase activity) from nonrecombinants (B-galactosidase activity), and pMC9 (a pBR322 plasmid carrying lacI to repress expression of foreign genes that might be detrimental to host cell and phage growth).

E.  Identification of Clones Containing Factor VIII-R Sequence

To screen the library for Factor VIII-R antigenic determinants producing clones, lambda gt11 recombinant phage were

plated on a lawn of E.coli Y1090 [ lacU169 proA⁺ lon araD139 strA supF [trpC22::Tn10] (pMC9)] and incubated at 42° C for 2.5 hours. This host was deficient in the lon protease, thereby reducing the degradation of expressed foreign protein. A nitrocellulose filter, previously saturated with 10 mM isopropyl thio-B-d-galactopyranoside (IPTG), an inducer of lacZ transcription, and dried, was overlaid on the plates. The plates were then removed to 37° C for 1.5 hours. The expression of foreign DNA inserts in lambda gt11 was under common control with lacZ transcription and, as such, was also induced. The position of the filter was marked with a needle, the filter was removed, washed in TBS buffer (20 mM tris, pH 7.5 and 500 mM NaCl), and then incubated in TBS plus 3% gelatin for 60 minutes at room temperature.

The filter was then incubated at room temperature overnight in a 1:100 dilution of monoclonal antibody, Zimmerman, PNA, 79:1648 (1982), directed against Factor VIII-R in a buffer consisting of 1% gelatin in TBS. The filter was then subjected to two washes of 10 minutes each in TBS buffer followed by addition of 20 ml of a 1:2000 dilution of horseradish peroxidase (HRP) coupled goat anti-mouse IgG (Kirkeguard & Perry, Gaithersburg, MD), followed by incubation for one hour at room temperature, and two washes of 10 minutes each in TBS. The filters were then incubated at room temperature in HRP color development solution as described in the Kirkeguard & Perry accompanying literature.

A 4-mm-diameter agar plug at the position of each of the color development signals was removed from the plates and incubated in 10 mM tris HCl, pH 7.5, and 10 mM MgSO₄ for at least one hour. Phage in this solution were replated on 90-mm plates at a density of approximately 10³ plaque-forming units (PFU) and rescreened as described above. This replating and screening process was repeated until all plaques on the plate produce a signal. These plaques identified recombinant clones which produce human Factor VIII-R.

Thus, this example describes experimental procedures which provide human Factor VIII-R essentially free of other proteins of human origin from a human tissue extract containing RNA coding for Factor VIII-R protein in a heterogeneous mixture with RNA for a broad library of other human proteins or poly-peptides.

WHAT IS CLAIMED IS:

1.  Human Factor VIII-R allelic variations, modifications and analogs thereof essentially free of other proteins of human origin.

2.  The Human Factor VIII-R according to Claim 1 in essentially pure form.

3.  A replicable expression vector capable, in a cell or cell-free self-replicating recombinant system, of expressing the human Factor VIII-R of Claims 1 or 2.

4.  A cell or cell-free self-replicating recombinant system transformed with the vector of Claim 3.

5.  The recombinant system of Claim 4 obtained by transforming an E.coli or B.subtilis.

6.  A recombinant system obtained by incorporating a cell of natural endogenous Factor VIII-R production with the expression vector of Claim 3.

7.  A process for producing human Factor VIII-R which process comprises:

a.  preparing a replicable expression vector capable of expressing the DNA sequence encoding human Factor VIII-R in a suitable self-replicating recombinant host system;

b.  transforming said host system to obtain a recombinant host system;

c.  maintaining said recombinant host system under conditions permitting expression of said Factor VIII-R encoding DNA sequence to produce human Factor VIII-R;

d.  recovering said human Factor VIII-R.

8.  The process of Claim 7 wherein preparing the replicable expression vector comprises the steps of preparing a ds-cDNA from a messenger RNA population containing messenger RNA encoding Factor VIII-R and incorporating the DNA from the ds-cDNA into replicable expression vectors.

9. The process of Claim 7 wherein the expression vector is a bateriophage.

10. The process of Claim 9 wherein the bacteriophage is lambda gt10 or lambda gt11.

11. The process of Claim 7 wherein recovering said human Factor VIII-R comprises reacting the proteins expressed by the recombinant host system with a reagent composition comprising at least one binding protein specific for Factor VIII-R, observing any detectable response therefrom and isolating the detected Factor VIII-R from the host system.

12. The process of Claim 11 wherein the specific binding protein is primary antibody.

13. The process of Claim 12 wherein the antibody is a polyclonal antibody.

14. The process of Claim 12 wherein the antibody is a monoclonal antibody.

15. The process of any of Claims 11-14 wherein the antibody is associated with a substance effective to provide a detectable response.

16. The process of Claim 11 wherein the reagent composition comprises a primary antibody which is specific for human Factor VIII-R and a secondary antibody which is specific for the primary antibody.

17. The process of Claim 16 wherein the primary and secondary antibodies are both polyclonal.

18. The process of Claim 16 wherein the secondary antibody is associated with a substance effective to provide a detectable response.

19. The process of either of Claims 15 or 18 wherein the substance effective to provide a detectable response comprises an isotopic or non-isotopic label.

20. The process of either of Claims 15 or 18 wherein the substance comprises an enzyme, its substrate and a reagent specifically responsive to the interaction of the enzyme and its substrate to provide a detectable response.

21. The process of Claim 20 wherein the enzyme is a peroxidase, the substrate is a peroxide and the reagent is a redox chromogen.

22. The process of Claim 7 wherein recovering said human Factor VIII-R comprises incubating the recombinant host system containing the ds-cDNA pool with.a labeled oligonucleotide probe sequencing for at least a portion of the Factor VIII-R protein such that the probe hybridizes with ds-cDNA encoding for Factor VIII-R and isolating and culturing the Factor VIII-R ds-cDNA-containing replicable expression vector so identified.

23. A composition comprising a therapeutically effective amount of human Factor VIII-R according to Claims 1 or 2 in admixture with a pharmaceutically acceptable carrier.

24. The composition according to Claim 23 suitable for parenteral administration.

25. The use of Factor VIII-R according to Claims 1 or 2 for treatment of disorders of hemastasis or for preparing pharmaceutical compositions useful in such treatment.

26. Human Factor VIII-R when made by the process of any of Claims 7-22.

0169562

5'    mRNA    3'
〰〰〰〰〰 AAAA

REVERSE
TRANSCRIPTASE

〰〰〰〰 AAAA
⎓⎓⎓⎓⎓ TTTT

HEAT DENATURATION

DNA POLYMERASE
(KLENOW)

SI NUCLEASE
SIZE FRACTIONATION

EcoRI METHYLASE

DNA POLYMERASE I
(KLENOW)

T₄ DNA LIGASE
EcoRI LINKERS

EcoRI DIGESTION
SIZE FRACTIONATION

Fig. 1

Fig. 2

## EUROPEAN SEARCH REPORT

Application number

| | DOCUMENTS CONSIDERED TO BE RELEVANT | | EP 85109326.0 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X,D | BLOOD, vol. 58, no. 1, July 1981, (New York)<br><br>L.W. HOYER "The Factor VIII Complex: Structure and Function" pages 1-13<br><br>   * Page 1; page 5, right column - page 6 *<br><br>-- | 1,2 | C 07 K 15/14<br>C 12 N 15/00<br>C 12 P 21/00<br>A 61 K 37/02<br>A 61 K 35/14<br>A 61 K 35/16<br>//C 12 R 1:19<br>C 12 R 1:125 |
| D,A | US - A - 4 361 509 (ZIMMERMAN et al.)<br><br>   * Abstract *<br><br>-- | 1,2 | |
| A | THE JOURNAL OF BIOLOGICAL CHEMISTRY, vol. 248, no. 11, June 10, 1973 (Baltimore, USA)<br><br>M.E. LEGAZ et al. "Isolation and Characterization of Human Factor VIII (Antihemophilic Factor)" pages 3946-3955<br><br>   * Summary *<br><br>-- | 1,2 | |
| D,A | PROCEEDINGS OF THE NATIONAL ACADEMY OF SCIENCES OF THE UNITED STATES OF AMERICA, vol. 80, March 1983, (Baltimore, USA)<br><br>R.A. YOUNG et al. "Efficient isolation of genes by using antibody probes" pages 1194-1198<br><br>   * Abstract *<br><br>---- | 7,9, 10 | |

**TECHNICAL FIELDS SEARCHED (Int. Cl.4)**

C 07 K
C 12 N
C 12 P
A 61 K

The present search report has been drawn up for all claims

| Place of search | Date of completion of the search | Examiner |
|---|---|---|
| VIENNA | 23-10-1985 | WOLF |